# FASCICULE DE BREVET EUROPEEN

(11) **EP 3 476 284 B1**
(45) Date de publication et mention de la délivrance du brevet: **04.12.2019**
(21) Numéro de dépôt: 18191006.8
(22) Date de dépôt: 27.08.2018
(51) Int. Cl.: B81B 3/00, B81B 7/00, A61B 5/0215, G01L 9/00

(54) **CAPTEUR MONOLITHIQUE INTÉGRÉ BIOCOMPATIBLE, NOTAMMENT POUR DISPOSITIF MÉDICAL IMPLANTABLE ACTIF**
BIOKOMPATIBLER INTEGRIERTER MONOLITHISCHER SENSOR, INSBESONDERE FÜR EINE AKTIVE IMPLANTIERBARE MEDIZINISCHE VORRICHTUNG
BIOCOMPATIBLE INTEGRATED MONOLITHIC SENSOR, NOTABLY FOR AN ACTIVE IMPLANTABLE MEDICAL DEVICE

(30) Priorité: 24.10.2017 FR 1760013
(43) Date de publication de la demande: 01.05.2019
(73) Titulaire: Mistic, 92130 Issy Les Moulineaux (FR)
(72) Inventeur: BOUTAUD, Bertrand, 92130 Issy les Moulineaux (FR)
(74) Mandataire: Dupuis-Latour, Dominique

(56) Documents cités:
- DE-A1- 19 839 122
- FR-A1- 2 947 629
- US-A1- 2011 314 922

## Description

L'invention concerne les capteurs monolithiques intégrés, en particulier ceux de ces capteurs qui comportent comme élément sensible un organe mobile sous l'effet d'une contrainte extérieure liée à la grandeur à mesurer.

Ces capteurs peuvent être de natures très variées : capteurs de pression où le milieu environnant exerce sur une membrane une pression déformant plus ou moins cette membrane, capteurs accélérométriques où une masse inertielle, intégrée ou rapportée, sollicite plus ou moins une structure déformable, capteurs microphoniques où les ondes acoustiques sont recueillies et transformées en vibrations mécaniques d'une membrane intégrée au capteur, etc.

Dans tous les cas, la variation de la grandeur à mesurer se traduit par une déformation géométrique, qui peut par exemple induire une variation de la distance relative entre deux éléments de transducteur, le transducteur comprenant généralement un élément mobile lié à la membrane ou autre organe déformable, et un élément fixe lié au corps du capteur.

Cette variation de distance est convertie au sein du capteur en une variation d'un paramètre électrique, mesurable par diverses techniques en elles-mêmes bien connues telles que mesure capacitive (les deux éléments de transducteur étant des électrodes en vis-à-vis d'un condensateur variable), mesure résistive (les électrodes venant établir des contacts successifs au fur et à mesure de la déformation de l'organe mobile), etc. La déformation géométrique peut également être directement convertie en une variation de potentiel électrique par le biais d'une couche piézoélectrique associée à l'organe déformable et déformée en même temps que celui-ci.

Les variations du paramètre électrique recueillies par ces divers moyens se retrouvent aux bornes de sortie du capteur, qui sont elles-mêmes reliées par exemple à un convertisseur analogique/numérique, à un processeur de données, un étage de filtrage, etc. du dispositif.

Un premier but de l'invention est de proposer pour les capteurs de ce type une structure entièrement monolithique permettant d'en accroitre le degré d'intégration, tout en conservant les qualités indispensables de robustesse mécanique, d'isolation électrique et de simplicité des processus de réalisation en ne faisant appel qu'à des techniques conventionnelles, bien maîtrisées et aisées à industrialiser à grande échelle.

Un autre but de l'invention est de proposer une telle structure de capteur qui soit utilisable dans le domaine médical, avec les contraintes propres à ce secteur.

L'invention s'applique en particulier très avantageusement - mais non exclusivement - à la réalisation de dispositifs médicaux implantables actifs (DMIA). Ces dispositifs peuvent en effet utiliser des capteurs du type précité, par exemple pour mesurer les variations instantanées de la pression sanguine, notamment de la pression cardiaque endocavitaire dans le cas des implants cardiaques, ou encore pour mesurer les ondes produites par les variations de l'accélération endocardiaque, paramètre qui est très étroitement corrélé à l'hémodynamique du patient et à la mécanique des battements cardiaques.

Les capteurs utilisés dans ces applications sont des capteurs réalisés sur substrat silicium, matériau qui présente l'inconvénient de ne pas être reconnu comme biocompatible.

De telles structures de capteur ne peuvent donc pas être implantées directement dans le corps humain en contact avec les fluides corporels et les tissus internes du patient. Elles nécessitent impérativement une encapsulation protectrice par une enveloppe en matériau biocompatible, avec la difficulté particulière que l'encapsulation doit permettre la déformation d'un élément sensible du capteur au niveau du matériau biocompatible. Cette déformation de l'enveloppe doit être ensuite transmise à la membrane (ou autre organe déformable) en silicium du capteur proprement dit pour détection par le transducteur. Cette transmission est réalisée par divers moyens mécaniques tels qu'une tige de liaison ou un fluide remplissant une cavité tampon entre le matériau biocompatible et la membrane en silicium.

Le FR 2 947 629 A1 décrit un capteur comprenant : un premier substrat en matériau conducteur avec une face externe et une face interne, comprenant : une région opérante déformable sous l'effet d'une sollicitation mécanique appliquée sur la face externe, et sur la face interne de la région opérante, un premier élément de transducteur, mobile sous l'effet de la déformation de la région opérante ; un second substrat en matériau conducteur avec une face externe et une face interne tournée vers la face interne du premier substrat, et comprenant une région opérante ; un second élément de transducteur apte à coopérer avec le premier élément de transducteur pour faire varier un paramètre électrique entre le premier et le second élément de transducteur sous l'effet de ladite sollicitation mécanique ; une liaison de solidarisation du premier substrat au second substrat, située à l'extérieur des régions opérantes ; une première borne de capteur, électriquement couplée au premier élément de transducteur ; et une seconde borne de capteur, électriquement couplée au second élément de transducteur, dans lequel, pour les deux substrats : la région opérante s'étend en direction transversale dans l'épaisseur du substrat d'une face à l'autre de celui-ci ; et dans lequel, pour le deuxième substrat : la région opérante est en forme d'ilot, physiquement isolé du reste du substrat, l'une des bornes de capteur étant électriquement reliée à l'ilot.

Le US 2008/0082005 A1 décrit une autre technique de capteur, qui utilise une antenne intégrée dans un substrat biocompatible et mobile dont la déformation modifie les paramètres LC de l'antenne, avec un circuit externe convertissant cette déformation en une variation de fréquence. Les dimensions nécessaires à une telle antenne déformable sont cependant un obstacle à une miniaturisation du capteur, et il est de plus nécessaire de protéger le capteur par des moyens externes tels que des filtres à l'encontre des champs magnétiques élevés que peut subir le dispositif lorsque le patient est soumis à un examen dans un appareil d'imagerie IRM. Ces structures de l'état de la technique sont toutes complexes, et sont loin d'être optimales tant en termes de coût d'industrialisation que de possibilités de miniaturisation.

L'invention propose de résoudre ces difficultés par une structure de capteur qui puisse être réalisée notamment (mais non nécessairement) en un matériau qui soit biocompatible, biostable et résistant à la corrosion. En d'autres termes, il s'agit de réaliser directement le capteur dans un matériau qui puisse être mis directement en contact avec les fluides et tissus corporels, et ceci pendant toute la durée de vie du dispositif implanté, typiquement pendant une dizaine d'années.

L'invention a également pour objet de proposer une telle structure de capteur qui puisse être réalisée par des technologies conventionnelles et bien maitrisées, donc avec de faibles coûts d'industrialisation.

Un autre but encore de l'invention est de proposer une structure de capteur qui autorise une miniaturisation extrême du composant. Cet aspect est particulièrement important dans le cas des dispositifs médicaux implantés, tout particulièrement certains dispositifs tels que les capsules autonomes endocavitaires dites *leadless,* qui sont entièrement implantées dans une cavité cardiaque, ainsi qu'en cas d'intégration dans une sonde cardiaque de diamètre très réduit.

À cet effet, l'invention propose un capteur comprenant, de manière en elle-même connue :
- un premier substrat en matériau conducteur avec une face externe et une face interne, comprenant une région opérante déformable sous l'effet d'une sollicitation mécanique appliquée sur la face externe et, sur la face interne de la région opérante, un premier élément de transducteur, mobile sous l'effet de la déformation de la région opérante ;
- un second substrat en matériau conducteur avec une face externe et une face interne tournée vers la face interne du premier substrat, et comprenant une région opérante ;
- un second élément de transducteur apte à coopérer avec le premier élément de transducteur pour faire varier un paramètre électrique entre le premier et le second élément de transducteur sous l'effet de ladite sollicitation mécanique ;
- une liaison de solidarisation du premier substrat au second substrat, située à l'extérieur des régions opérantes ;
- une première borne de capteur, électriquement couplée au premier élément de transducteur ; et
- une seconde borne de capteur, électriquement couplée au second élément de transducteur.

De façon caractéristique de l'invention, pour au moins l'un des substrats :
- la région opérante s'étend en direction transversale dans l'épaisseur du substrat d'une face à l'autre de celui-ci ;
- la région opérante est en forme d'ilot de contour fermé, physiquement et électriquement isolé du reste du substrat, l'une des bornes de capteur étant électriquement reliée à l'ilot ;
- il est prévu entre la région opérante et le reste du substrat une interface comprenant au moins une couche latérale périphérique en matériau électriquement isolant propre à assurer à la fois un assujettissement mécanique de la région opérante au substrat et une isolation électrique entre la région opérante et le substrat, la couche latérale périphérique étant monolithique avec le reste du substrat et avec la région opérante, enveloppant en direction latérale la région opérante sur toute sa périphérie, et s'étendant en direction transversale sur l'épaisseur du substrat.

Selon diverses caractéristiques subsidiaires avantageuses :
- le matériau du premier substrat et du second substrat est un matériau appartenant à la classe des matériaux biocompatibles, biostables et résistants à la corrosion, notamment le titane ;
- le matériau électriquement isolant de la couche latérale périphérique est un oxyde du matériau du substrat métallique ;
- la région opérante déformable du premier substrat est située sur une partie d'épaisseur réduite du premier substrat ;
- la région opérante du second substrat est une région située en vis-à-vis de la région opérante déformable du premier substrat, le second élément de transducteur est un élément disposé sur la face interne de la région opérante du second substrat en vis-à-vis du premier élément de transducteur, et les premier et second éléments de transducteur sont des éléments de l'un parmi un transducteur capacitif, un transducteur résistif, et un transducteur inductif ;
- la région opérante déformable du premier substrat comprend une couche piézoélectrique, et les premier et second éléments de transducteur sont des surfaces opposées de la couche piézoélectrique, comprenant une surface en contact avec la face interne de l'un des substrats, et une surface libre tournée à l'intérieur du capteur vers la face interne de l'autre substrat ;
- le capteur comporte en outre un composant électronique incorporé au capteur et comprenant une première borne de composant et une seconde borne de composant, et ledit au moins un des substrats comprend : sur sa face interne, un évidement logeant le composant électronique ; une autre interface, comportant au moins une autre couche latérale périphérique définissant un autre ilot isolé de l'ilot et du reste du substrat ; et une liaison électrique de la première borne de composant à l'autre ilot, et une liaison électrique de la seconde borne de composant au reste du substrat ou à l'ilot ;
- la liaison de solidarisation est une liaison soudée des premier et second substrats entre eux, ou bien elle comprend entre le premier substrat et le second substrat une couche intercalaire de scellement, notamment en un matériau électriquement conducteur apte à assurer une continuité électrique entre le premier et le second substrat ;
- ledit au moins un des substrats comprend sur sa face externe une couche isolante de surface, avec dans la région de l'ilot au moins une zone de contact mise à nu ou laissée à nu ;
- dans ce dernier cas, la zone de contact peut être proéminente en direction transversale par rapport au reste de la face externe du substrat, ou bien il peut être prévu deux zones de contact avec deux zones proéminentes respectives mises à nu ou laissées à nu, l'une dans la région de l'îlot et l'autre dans la région du reste du substrat, et entre les deux zones proéminentes, dans la région de la couche interne latérale périphérique, une zone en retrait avec une couche isolante.

On va maintenant décrire un exemple de réalisation de la présente invention, en référence aux dessins annexés où les mêmes références désignent d'une figure à l'autre des éléments identiques ou fonctionnellement semblables.
La Figure 1 est une vue en coupe transversale de traversées permettant la réalisation d'un capteur selon l'invention, montrant diverses variantes de réalisation possibles.
Les Figures 2 et 3 sont des vues, respectivement de dessus et en coupe transversale, illustrant un premier procédé de réalisation de traversées telles que celles illustrées Figure 1, selon différentes configurations possibles, dans une étape initiale après gravure du substrat et avant oxydation de celui-ci.
La Figure 4 est une vue de dessus illustrant les phases successives (a) à (c) de l'étape d'oxydation du premier procédé précité, au niveau de l'un des ponts de matière visibles sur les Figures 2 et 3.
La Figure 5 est une vue en coupe transversale de la structure de la Figure 3, après achèvement complet de l'étape d'oxydation.
La Figure 6 est une vue en coupe transversale illustrant les phases successives (a) à (c) de réalisation d'un contact électrique sur l'élément traversant, selon une première technique possible de prise de contact.
La Figure 7 est une vue en coupe transversale illustrant les phases successives (a) à (c) de réalisation d'un contact électrique sur l'élément traversant, selon une seconde technique possible de prise de contact.
La Figure 8 est une vue en coupe transversale illustrant, selon deux variantes possibles, les différentes phases (a) à (d) de réalisation d'une traversée par le premier procédé précité, dans le cas où il est prévu un amincissement du substrat dans la région de la traversée.
Les Figures 9 à 12 sont des vues en coupe transversale illustrant les différentes étapes successives d'un second procédé précité de réalisation d'une traversée.
Les Figures 13a et 13b sont des vues en coupe transversale illustrant, selon deux possibilités de mise en œuvre respectives, une variante du procédé des Figures 9 à 12, au stade du procédé où les tranchées borgnes viennent d'être gravées
Les Figures 14a et 14b sont homologues des Figures 13a et 13b, après achèvement de l'étape d'oxydation contrôlée du matériau du substrat.
La Figure 15 est une vue en coupe transversale d'un capteur selon l'invention.
La Figure 16 est une vue en coupe transversale d'une variante de réalisation du capteur de la Figure 15.
La Figure 17 est une vue en coupe transversale d'un capteur tel que celui illustré Figure 15, incorporant en outre en son sein un composant électronique rapporté.
La Figure 18 est une vue en coupe transversale d'une variante de réalisation du capteur de la Figure 15, avec une couche isolante structurée de surface sur l'une de ses faces extérieures.
La Figure 19 est une vue en coupe transversale d'une variante de réalisation du capteur de la Figure 18.
La Figure 20 est une vue en coupe transversale d'une autre variante de réalisation du capteur de la Figure 18.
La Figure 21 est une vue en coupe transversale d'une variante de réalisation du capteur de la Figure 19.

On va en premier lieu exposer divers exemples de traversées permettant la réalisation d'un capteur selon l'invention, ainsi que deux procédés de réalisation de telles traversées.

### Structure de traversée

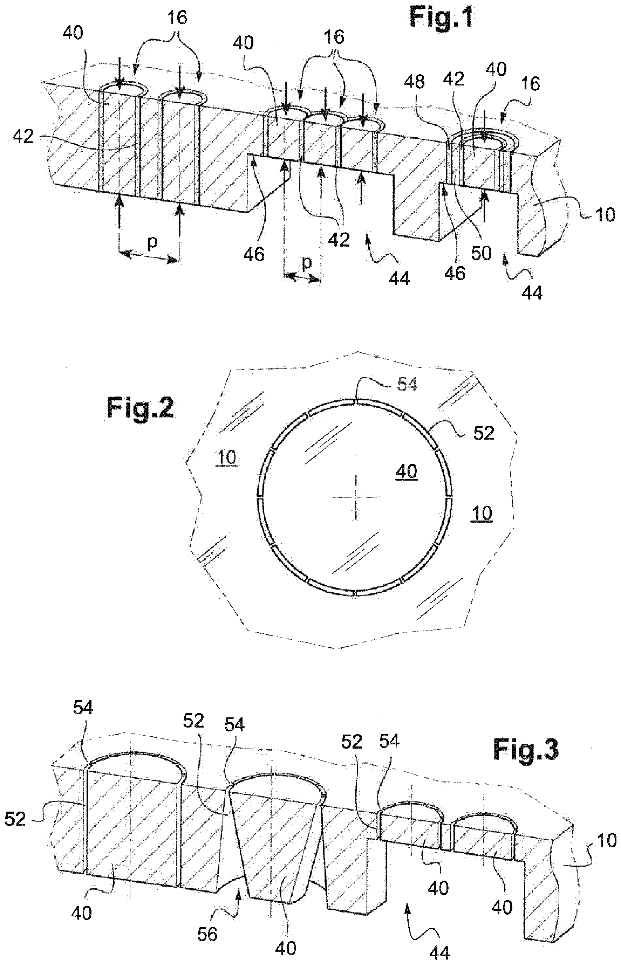

La Figure 1 est une vue en coupe transversale du substrat 10 montrant plusieurs types de traversées pour la réalisation d'un capteur selon l'invention, avec les variantes suivantes :
- à gauche de la figure, réalisation de deux traversées 16 sur la totalité de l'épaisseur du substrat 10 (épaisseur pouvant varier, typiquement mais de manière non limitative, entre 50 µm et 500 µm), ces deux traversées étant voisines mais non attenantes ;
- au centre, réalisation de trois traversées 16 attenantes, celles-ci étant en outre réalisées sur une région d'épaisseur réduite du substrat ;
- à droite, réalisation d'une traversée simple sur une région d'épaisseur réduite du substrat, avec une structure procurant une double isolation et/ou permettant l'intégration éventuelle d'un composant capacitif intégré de filtrage ou de découplage.

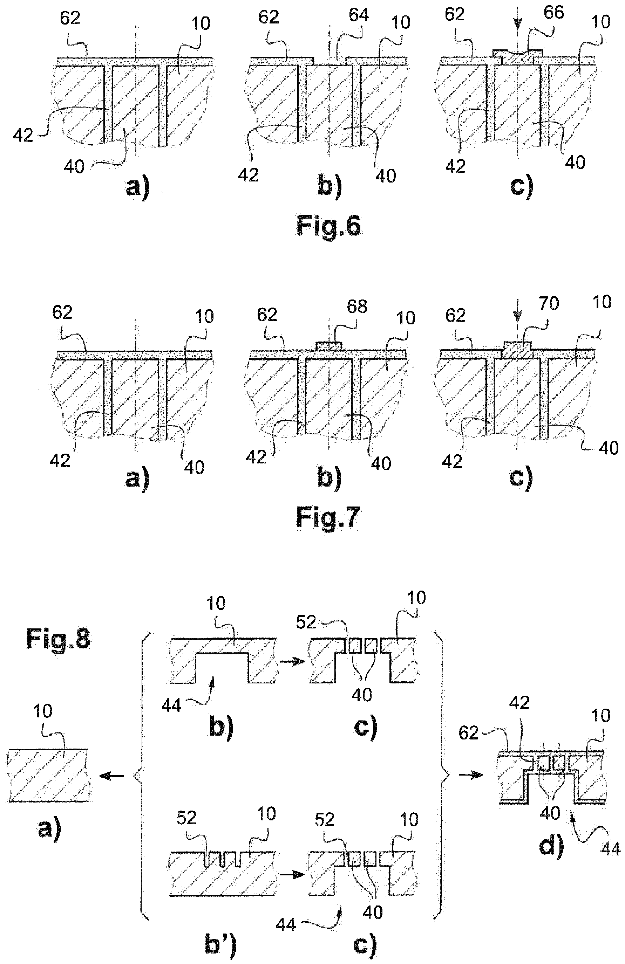

Sur les figures, les flèches verticales schématisent les emplacements où pourront être réalisées d'un côté et de l'autre du substrat les prises de contact sur l'élément traversant de chaque traversée 16, selon des techniques en elles-mêmes connues qui seront brièvement décrites par la suite, notamment en référence aux Figures 6 et 7.

Chaque traversée 16 comprend un élément traversant métallique 40, qui est un élément formé dans le matériau du substrat et s'étendant en direction transversale dans l'épaisseur du substrat, d'une face à l'autre de celui-ci. Dans le sens latéral, l'élément traversant 40 est agencé comme un élément en forme d'ilot de contour fermé, physiquement et électriquement isolé du substrat.

On notera que dans la présente description le qualificatif "transversal" indique une direction correspondant à l'épaisseur du substrat, donc perpendiculaire à la surface de celui-ci, tandis qu'une direction "latérale" qualifiera une direction s'étendant suivant l'étendue du substrat, autrement dit une direction radiale par rapport à un axe transversal de la traversée.

En ce qui concerne le contour périphérique fermé de l'ilot définissant l'élément de traversée, ce contour peut être de toute forme : circulaire, polygonale (rectangulaire) ou quelconque, dès lors que par son caractère fermé elle isole complètement l'ilot du reste du substrat, physiquement et électriquement (le terme "périphérique" devant être entendu comme qualifiant un ilot structurellement séparé et électriquement isolé du reste du substrat).

Par ailleurs, la génératrice définissant le contour de l'ilot ne s'étend pas nécessairement perpendiculairement au substrat : par exemple, le contour peut être cylindrique de révolution, mais aussi cylindrique quelconque, ou également conique (voir notamment Figure 3, au centre), pyramidal, ou de toute forme quelconque.

La traversée comprend en outre une interface de couplage de l'élément traversant 40 au reste du substrat 10, qui assure à la fois l'assujettissement mécanique de cet élément traversant au substrat et l'isolation électrique entre élément traversant et substrat.

De façon caractéristique, cette interface de couplage est assurée par une couche latérale périphérique 42 en matériau électriquement isolant, enveloppant en direction latérale l'élément traversant 40 sur toute sa périphérie et s'étendant, en direction transversale, sur l'épaisseur du substrat.

Les techniques de réalisation que l'on décrira plus loin permettent en particulier de réaliser un ensemble monolithique comprenant à la fois le substrat, l'élément traversant et la couche latérale, ensemble dans lequel cette couche latérale assure, essentiellement et directement à la fois i) l'assujettissement mécanique de l'élément traversant au substrat et ii) l'isolation électrique entre l'élément traversant et le substrat, grâce à une jonction (mécanique) directe et latérale de l'élément traversant 40 par rapport au substrat 10.

Très avantageusement, le matériau de la couche latérale périphérique 42 est un oxyde du métal constituant le substrat 10, notamment de l'oxyde de titane TiO₂ : en effet, le titane et son oxyde sont des matériaux qui présentent les propriétés avantageuses de biocompatibilité, de biostabilité et de résistance à la corrosion qui les rendent particulièrement appropriés à un très grand nombre d'applications, notamment pour les dispositifs médicaux actifs en contact avec des tissus ou fluides corporels, tout particulièrement les dispositifs médicaux implantables.

Dans l'exemple illustré à gauche de la Figure 1, deux traversées 16 semblables ont été réalisées de la même manière, avec un pas d'entraxe p qui peut être réduit d'autant que l'emprise en direction latérale est celle du seul élément traversant 40 avec sa couche latérale 42, sans aucun débordement périphérique.

Comme illustré en partie centrale de la Figure 1, la(les) traversée(s) 16 peu(ven)t être réalisées sur une partie d'épaisseur réduite 44 du substrat, avec creusement de cette partie réduite depuis la face inférieure du substrat 10 selon des techniques qui seront décrites par la suite notamment en référence à la Figure 8.

Toujours dans l'exemple illustré au centre de la Figure 1, on a représenté une pluralité de traversées 16 formées avec leurs couches latérales périphériques 42 adjacentes les unes aux autres (c'est-à-dire qu'une couche isolante d'oxyde peut être commune à deux traversées adjacentes dans la région où celles-ci sont les plus proches), ce qui permet de réduite encore plus le pas d'entraxe p entre traversées adjacentes.

Dans le cas où une partie d'épaisseur réduite 44 est formée, il peut y avoir, ou non, un retrait ou épaulement 46 entre la couche latérale périphérique 42 et le bord latéral de la partie d'épaisseur réduite 44.

À droite de la Figure 1 est représenté un exemple de traversée simple comprenant deux couches latérales périphériques 42,48 concentriques (le terme de "concentrique" étant entendu au sens le plus général, c'est-à-dire qu'une couche 48 extérieure entoure complètement une couche intérieure 42, les contours de ces couches n'étant pas nécessairement coaxiaux, ni même circulaires).

Le fait d'avoir plusieurs couches latérales périphériques permet en particulier de maximiser l'isolation électrique entre l'élément traversant 40 et le reste du substrat 10.

Cette configuration permet aussi d'éloigner physiquement l'élément traversant 40 et le reste du substrat 10, ce qui peut être intéressant dans certaines applications telles que les applications radiofréquence, où il est important de réduire le couplage électromagnétique entre structures voisines. En particulier (et aussi bien pour ce mode de réalisation que pour les autres) l'élément traversant 40 peut non seulement constituer une traversée, mais également une antenne émettrice/réceptrice radiofréquence isolée et éloignée du reste du substrat 10. Dans un tel cas, l'élément 40 pourra adopter toute forme de géométrie d'antenne connue, telle que boucle, zigzag, spirale, fourche, etc. On pourra se référer notamment au EP 2 873 437 A1, qui décrit un tel type de composant RF intégré à un substrat et relié à une traversée monolithique réalisée au travers de ce substrat.

Cette configuration permet également, par un choix approprié des dimensions de la couche latérale périphérique, de réaliser une structure intégrée de condensateur de filtrage ou de découplage pouvant être associé à la traversée : en effet, l'alternance des couches concentriques métal/oxyde/ métal correspond à une structure armature/diélectrique/armature d'un condensateur, la couche intercalaire faisant office de diélectrique. Tel est le cas des couches respectives :
- 40/42/10 (comme à gauche de la Figure 1), ou
- 40/42/50 et 50/48/10 (comme à droite de la Figure 1).

Les paramètres de ce condensateur (capacité, tension de claquage) sont modulables par un choix approprié de l'épaisseur d'isolant et des dimensions de l'élément traversant (surface du contour périphérique et longueur en direction transversale).

Ces paramètres peuvent être choisis en fonction du but recherché : soit pour réaliser un couplage contrôlé avec le substrat (par exemple à des fins de filtrage), soit au contraire pour découpler le plus possible les traversées d'avec le substrat. Dans ce dernier cas, il peut notamment être avantageux d'augmenter le nombre des interfaces concentriques (comme à droite de la Figure 1, où ces interfaces concentriques sont au nombre de deux) car dans ce cas les capacités (indésirables) se trouvent électriquement en série, ce qui divise d'autant la capacité de couplage globale entre le substrat 10 et l'élément traversant principal 40 - notamment lorsque celui-ci assure aussi une fonction d'antenne.

### Premier exemple de procédé de réalisation d'une structure de traversée

En référence aux Figures 2 à 8, on va maintenant décrire un premier procédé permettant d'obtenir une structure de traversée selon les différentes variantes illustrées Figure 1.

La première étape de ce procédé consiste, comme illustré aux Figures 2, 3 et 4(a), à graver le substrat 10 en creusant sur son épaisseur une tranchée débouchante 52. En direction latérale, le contour de cette tranchée définit un ilot central correspondant à l'élément traversant 40 de la traversée à réaliser.

La tranchée débouchante 52 s'étendant sur l'épaisseur du substrat de part en part de celui-ci, pour pouvoir maintenir en place l'élément central 40 il est prévu de laisser subsister de minces ponts radiaux 54 de matière non gravée. Le nombre et la configuration de ces ponts ou bras de matière non gravée peut être compris entre 1 et un nombre aussi élevé que souhaitable, ces ponts pouvant être symétriques ou non, et régulièrement répartis ou non.

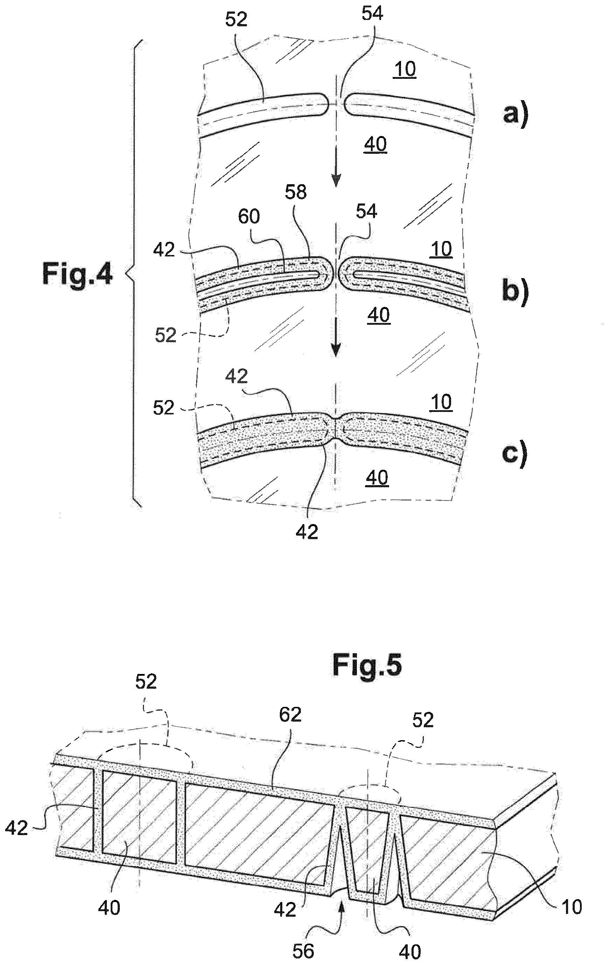

À ce stade, l'élément central est maintenu assujetti au reste du substrat par les ponts 54, mais il n'est pas isolé électriquement du substrat puisque les ponts laissent subsister un chemin conducteur. Il n'y a pas non plus d'étanchéité au travers de la structure entre l'élément central 40 et le reste du substrat 10, puisque les secteurs gravés de la tranchée 54 débouchent de part et d'autre de la surface et laissent subsister un volume intérieur libre, visible notamment sur la représentation agrandie de la Figure 4(a).

La Figure 3 illustre diverses variantes possibles de réalisation de la tranchée débouchante 52.

À gauche sur la Figure 3, la tranchée 52 est une tranchée dont les deux parois en vis-à-vis sont parallèles, la tranchée s'étendant en outre sur toute l'épaisseur du substrat.

Au centre de la Figure 3, on a représenté une variante où les parois en vis-à-vis de la tranchée ne sont pas parallèles, la tranchée présentant une plus grande largeur en 56 du côté de l'une des faces du substrat (ce non-parallélisme peut être notamment introduit par le procédé de fabrication qui génère un angle de pénétration, cela pouvant être vrai dans toutes les configurations). De ce fait, les ponts de matière 54 ne s'étendent plus que sur une fraction de l'épaisseur du substrat, à la différence de la variante représentée à gauche sur la Figure 3.

À droite sur la Figure 3, on a représenté une autre variante où la traversée est réalisée dans une partie d'épaisseur réduite 44 du substrat, ce qui permet, ici encore, de réduire la longueur en direction transversale des ponts de matière 54 (et également celle de l'élément central 40). Cette variante peut être réalisée de deux façons différentes :
- dans une première sous-variante, le substrat est d'abord aminci dans la région 44 pour lui donner une épaisseur réduite, puis la tranchée 52 est réalisée comme décrit précédemment, c'est-à-dire par creusement d'une tranchée traversante débouchant dans la face inférieure de la région amincie 44 ;
- dans une seconde sous-variante, une tranchée borgne est creusée dans le substrat dans une fraction de son épaisseur, puis le substrat est aminci pour réaliser la région 44 d'épaisseur réduite, sur une profondeur suffisante pour rejoindre la tranchée borgne 52 qui avait été creusée depuis l'autre côté du substrat et rendre ainsi cette dernière traversante.

Le creusement de la tranchée traversante 52 a ainsi permis de détourer l'ilot central correspondant à l'élément traversant 40, ce dernier étant maintenu en place par les ponts de matière 54. Cette situation correspond à celle de la Figure 4(a).

L'étape suivante, illustrée aux Figures 4(b), 4(c) et 5, consiste à réaliser simultanément l'isolation électrique et l'étanchéité de l'élément central 40 par une étape d'oxydation thermique contrôlée du substrat.

Cette étape va produire, concurremment :
- en profondeur, la croissance d'un front d'oxyde isolant consommant le métal du substrat (celui de l'élément central 40 comme celui du reste du substrat 10), comme illustré en 58 sur la Figure 4(b), où l'emplacement initial de la tranchée avant oxydation est représenté en tiretés en 52 ; et
- en surface, la croissance d'une épaisseur d'oxyde sur les parois de la tranchée 52, comme illustrée en 60 sur la Figure 4(b).

La poursuite de l'oxydation contrôlée va entrainer un comblement progressif du volume intérieur libre subsistant entre les deux parois en vis-à-vis de la tranchée 52, jusqu'à comblement complet de ce volume libre, comme illustré sur la Figure 4(c).

Par ailleurs, la dimension périphérique des bras de liaison 54 a été choisie suffisamment faible pour que, une fois la phase de comblement complet achevée, le métal du pont de matière 54 soit entièrement transformé en oxyde, comme illustré également sur la Figure 4(c). Les deux fronts en vis-à-vis de part et d'autre du pont de matière 54 se sont rejoints, isolant ainsi électriquement, et rendant étanche, l'élément central 40 vis-à-vis du reste du substrat 10.

On a illustré sur la gauche de la Figure 5, en coupe transversale, la structure obtenue dans le cas d'une tranchée de largeur constante (configuration correspondant à celle illustrée sur la gauche de la Figure 3).

On a illustré sur la droite de la Figure 5 la structure obtenue dans le cas où l'on avait creusé une tranchée de largeur variable (configuration correspondant à celle illustrée au centre de la Figure 3) : le comblement de la tranchée ne se fait alors que sur une partie de l'épaisseur du substrat, choisie pour procurer le niveau requis à la fois d'assujettissement mécanique de l'élément central 40 au substrat 10 et d'isolation électrique de cet élément traversant 40 par rapport au substrat 10. Si la tenue mécanique n'est pas suffisante, l'oxydation thermique peut être poursuivie jusqu'à remplir la cavité subsistante 56 sur toute sa profondeur, ou bien cette dernière sera remplie par dépôt d'un matériau tiers, isolant ou conducteur. L'étape suivante consiste à réaliser une prise de connexion sur l'élément central 40.

En effet, celui-ci est isolé électriquement de tous côtés, notamment par la couche supérieure d'oxyde 62, et de même du côté opposé par la couche d'oxyde inférieure. Il est donc nécessaire, au niveau de l'élément traversant 40, de réaliser une continuité électrique entre les deux faces du substrat pour constituer la liaison électrique de la traversée.

La prise de connexion peut (dans ce mode de réalisation comme dans tous les autres) être réalisée sur l'une et l'autre des deux faces - supérieure et inférieure - de l'élément traversant central 40, avec report ou dépôt d'un élément de liaison conducteur (fil rapporté, piste en surface du substrat, etc.) destiné à assurer une liaison électrique à des éléments, circuits ou composants distants situés de part et d'autre du substrat. Mais la prise de connexion peut être réalisée sur une face seulement de l'élément traversant, l'autre face de l'élément traversant étant une face active directement utilisable, par exemple pour constituer une électrode surfacique plaquée sur une face d'un boitier de dispositif, ou encore au niveau d'un capteur intégré à un dispositif. Cette configuration est particulièrement avantageuse pour la réalisation d'un dispositif implantable où cette électrode surfacique est destinée à venir en contact avec un tissu du patient porteur du dispositif.

Dans tous les cas, il convient de mettre à nu ou de laisser à nu chacune des faces supérieure et inférieure de l'élément traversant central 40.

Une première solution consiste, avant l'oxydation, à déposer un matériau inhibiteur d'oxydation tel que par exemple nitrure de titane, nitrure de silicium, tungstène, platine, niobium ou palladium ou toute combinaison de ces matériaux, sur une épaisseur de quelques dizaines à centaines de nanomètres, dans les régions de la surface du substrat où l'on ne souhaite pas voir croitre l'oxyde. Le contact électrique est alors directement obtenu après oxydation et élimination de la couche inhibitrice, avec éventuellement superposition ultérieure d'une couche supplémentaire d'un matériau métallique tel qu'or, platine, palladium, niobium, iridium ou toute combinaison de ces matériaux.

Une autre solution, illustrée Figure 6, consiste après achèvement de l'oxydation (Figure 6(a)) à éliminer localement la couche d'oxyde, par exemple par gravure plasma ou laser, jusqu'à mettre à nu la surface du matériau métallique de l'élément central 40, comme illustré en 64 sur la Figure 6(b).

La zone de contact électrique ainsi exposée peut, ici encore, être optimisée par dépôt, comme illustré en 66 sur la Figure 6(c), d'une couche métallique additionnelle en un matériau tel qu'or, platine, palladium, niobium, iridium ou toute combinaison de ces matériaux. Cette couche métallique supplémentaire peut, en direction latérale, soit être confinée à la région 64 sans oxyde, soit déborder de celle-ci et recouvrir l'oxyde au-delà de la périphérie de la zone 64 (comme illustré en Figure 6(c)).

Une autre possibilité encore de réalisation de la prise de contact est illustrée Figure 7.

Après l'étape d'oxydation du substrat (Figure 7(a)), une couche d'un matériau approprié, tel qu'or, platine palladium, niobium, iridium ou toute combinaison de ces matériaux, est déposée au-dessus de la zone où l'on souhaite réaliser le contact, comme illustré en 68 sur la Figure 7(b). Un traitement thermique engendre alors une diffusion de ce matériau dans l'oxyde, comme illustré en 70 sur la Figure 7(c), ce qui a pour effet de rendre cet oxyde conducteur dans la zone sous-jacente.

Ces différentes techniques de réalisation de prise de contact sont en elles-mêmes connues et ne seront pas décrites plus en détail. Elles peuvent être mises en œuvre de la même façon de l'autre côté du substrat, de manière à définir une continuité électrique entre les deux faces, interne et externe, de l'élément traversant et réaliser ainsi la liaison électrique (ou chaque liaison électrique) de la traversée.

La Figure 8 illustre de façon schématique la réalisation d'une traversée sur une zone d'épaisseur réduite 44 du substrat, selon les deux variantes qui avaient été décrites plus haut en ce qui concerne le creusement de la tranchée : creusement préalable de la partie d'épaisseur réduite 44, puis de la tranchée traversante 52 (Figures 8(a) à 8 (c) ; ou creusement préalable de tranchées borgnes 52 puis creusement du substrat pour obtention de la partie d'épaisseur réduite 44 jusqu'à atteindre les tranchées 52 et les rendre traversantes (Figures 8(a), 8(b') et 8(c')).

Ces étapes selon l'une ou l'autre variante sont ensuite suivies de l'étape d'oxydation du substrat (Figure 8(d)) permettant d'obtenir la structure voulue pour la réalisation de traversées sur la région d'épaisseur réduite 44 du substrat.

### Second exemple de procédé de réalisation d'une structure de traversée

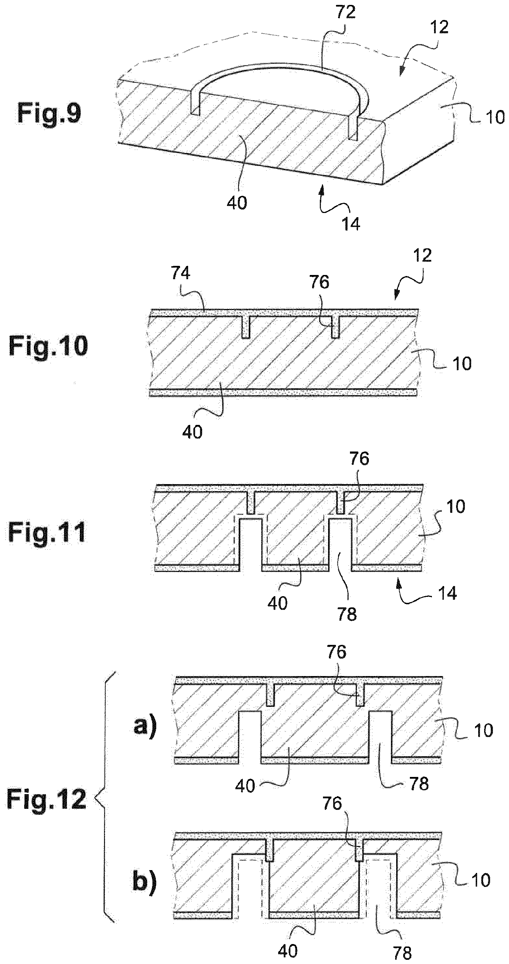

En référence aux Figures 9 à 12, on va maintenant décrire un second procédé permettant d'obtenir une structure de traversée.

La première étape, illustrée Figure 9, consiste à détourer un ilot (correspondant à l'élément traversant de la traversée à réaliser) par creusement dans le matériau du substrat, depuis une première face de celui-ci, par exemple la face supérieure 12, d'une tranchée borgne 72 s'étendant en direction transversale sur une fraction de l'épaisseur du substrat 10. En direction latérale, cette tranchée s'étend sur toute la périphérie de l'ilot central 40 - c'est-à-dire que la tranchée 72 ne laisse pas subsister de ponts de matière, à la différence de la tranchée 52 illustrée Figure 2 du procédé précédent.

L'étape suivante, illustrée Figure 10, consiste à procéder à une oxydation contrôlée du matériau du substrat, incluant une oxydation des parois latérales de la tranchée jusqu'à comblement de tout ou partie du volume intérieur libre de celle-ci par croissance de l'oxyde.

À l'issue de cette étape d'oxydation, le substrat comporte sur l'une et l'autre de ses faces une couche de revêtement d'oxyde 74 (typiquement de quelques micromètres d'épaisseur) s'étendant, du côté de la face supérieure 12, le long de la tranchée 76 comblée totalement (comme illustré

Figure 10) ou seulement partiellement. Dans le cas d'un remplissage partiel, de manière optionnelle le remplissage peut être rendu complet par le dépôt ultérieur d'une couche de remplissage isolante ou conductrice, de manière à notamment renforcer mécaniquement la structure.

L'étape suivante, illustrée Figure 11, consiste à creuser une gorge périphérique 78 depuis l'autre face du substrat, à savoir la face inférieure 14 dans l'exemple illustré. Cette gorge périphérique 78 peut être creusée sur une profondeur lui permettant d'atteindre la tranchée oxydée 76 et ainsi isoler complètement l'élément traversant central 40.

Pour éviter qu'une surgravure n'endommage l'oxyde isolant de la tranchée comblée 76, une variante avantageuse, illustrée Figure 11, consiste à arrêter le creusement de la gorge 78 légèrement avant d'atteindre la tranchée comblée 76 puis à achever la gravure par une attaque chimique plus sélective, illustrée en tiretés sur la Figure 11, cette attaque sélective attaquant essentiellement le substrat métallique 10 mais très peu l'oxyde de la tranchée comblée 76.

Toutefois, étant donné que les tolérances de profondeur de gravure entre le bord et le centre d'un même *wafer* portant un très grand nombre de composants distincts, certains endroits peuvent se trouver gravés plus profondément que d'autres.

Pour pallier cet inconvénient, et éviter une surgravure substantielle qui élargirait excessivement, notamment en direction latérale, les dimensions voulues, il est possible, comme illustré Figure 12, de décaler latéralement la gravure de la gorge 78 par rapport à la position de la tranchée comblée 76.

Ce décalage en direction latérale peut être réalisé soit vers l'intérieur soit, comme illustré Figure 12(a), vers l'extérieur, la profondeur de la gorge 78 gravée pouvant être inférieure, égale ou supérieure au niveau du fond de la tranchée comblée 76 située en vis-à-vis.

Dès lors, comme illustré en tiretés sur la Figure 12(b), une gravure isotropique du métal, chimique ou physique, se propageant dans toutes les directions permet dans toutes les configurations d'atteindre l'oxyde de la tranchée comblée 76 et d'isoler électriquement la partie centrale traversante 40 par rapport au reste du substrat. La profondeur de la gravure isotropique du métal est typiquement de l'ordre de 15 à 20 µm.

En effet, les tolérances d'alignement entre face avant et face arrière d'un même *wafer* sont très homogènes et très faibles (typiquement de 1 à 5 µm) quelle que soit la position du composant sur le *wafer,* ce qui réduit la profondeur de gravure isotropique nécessaire.

L'avantage de cette dernière variante est sa moindre sensibilité aux tolérances de profondeur de gravure, puisqu'il suffit d'atteindre une proximité minimale par rapport à l'oxyde de la tranchée comblée 76 pour compenser les variations dimensionnelles liées aux tolérances de fabrication.

De façon générale, et quelle que soit la variante de réalisation mise en œuvre, on notera que la couche isolante d'oxyde présente sur chaque face du substrat n'a plus aucune fonction ni électrique ni mécanique. Dès lors, cette couche d'oxyde peut être gravée totalement ou sélectivement pour mettre à nu le métal en surface du substrat. Le métal mis à nu peut être utilisé à diverses fins telles que contact électrique avec le substrat, soudure de la pièce réalisée sur un autre élément du dispositif, intégration d'une puce de circuit intégré, etc.

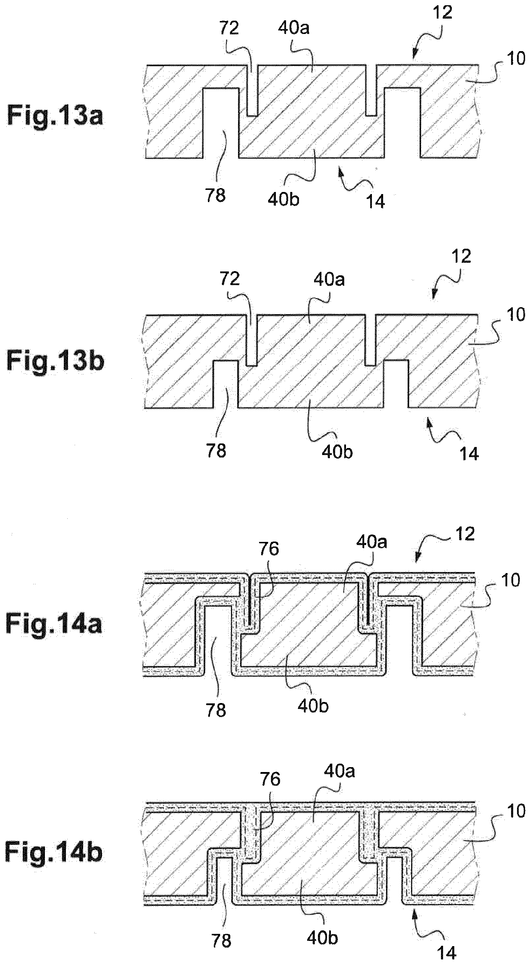

Les Figures 13a, 113b, 14a et 14b sont des vues en coupe transversale illustrant, selon deux possibilités différentes de mise en oeuvre, une variante du procédé des Figures 9 à 12, respectivement au stade du procédé où les tranchées borgnes 72 et 78 viennent d'être gravées (Figures 13a et 13b) et après achèvement de l'étape d'oxydation contrôlée du matériau du substrat.

Dans cette variante, où une tranchée 72 gravée sur une face est décalée par rapport à une tranchée 78 gravée sur l'autre face, l'isolation électrique entre les gravures (et donc entre l'ilot central et le reste du substrat) est établie par une oxydation 76 de l'interstice entre ces deux tranchées. Ceci peut être réalisé soit par gravure des deux tranchées 72, 78 décalées sur les faces opposées avec chevauchement vertical (Figure 13a), soit par gravure des deux tranchées 72, 78 décalées sur les faces opposées et en proximité verticale (Figure 13b).

L'ordre de réalisation des gravures 72, 78 sur une face puis l'autre est indifférent. Les tranchées peuvent être décalées dans des sens différents (décalage interne, décalage externe, décalage à cheval). Chacune des tranchées 72, 78 définit un ilot respectif 40a, 40b et ces deux ilots, lorsqu'ils seront électriquement isolés ensemble du reste du substrat, formeront l'élément conducteur central 40 de la traversée.

La profondeur de gravure des tranchées doit être suffisante de part et d'autre pour définir une séparation très fine entre les deux (dont la dimension peut varier, typiquement mais de manière non limitative, entre 0,5 µm et 25 µm). Elles peuvent être soit en chevauchement (Figure 13a) soit en proximité (Figure 13b).

Le décalage définissant l'interstice entre les deux tranchées sera suffisamment fin pour que cet interstice soit intégralement oxydé (comme en 76 sur les figures), devenant ainsi électriquement isolant.

Dans la mise en œuvre illustrée Figure 14b, l'une au moins des deux tranchées (la tranchée 72 des Figures 14a et 14b) peut être suffisamment étroite pour que son volume soit intégralement rempli par l'oxyde pendant l'oxydation thermique (comme en 76 Figure 14b). Si ce n'est pas le cas (comme en Figure 14a), le volume résiduel libre de la (des) tranchée(s) oxydée(s) peut être comblé - ou non - par le dépôt ultérieur d'un autre matériau, de manière à rigidifier la structure.

### Structure de capteur selon l'invention

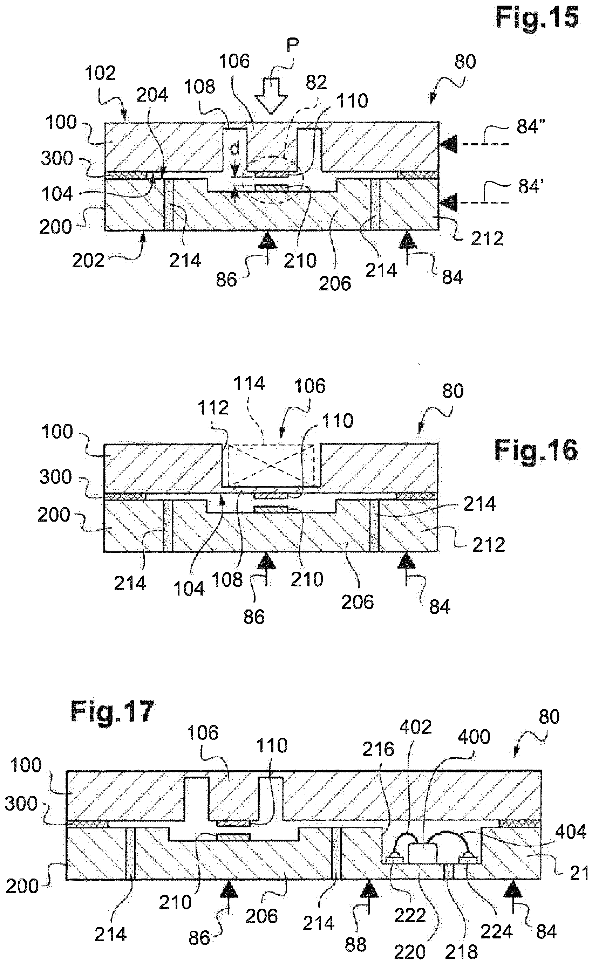

On va maintenant décrire une structure de capteur selon l'invention, en référence aux Figures 15 à 21 qui en illustrent diverses variantes de réalisation.

L'idée de base de l'invention consiste à tirer profit des excellentes performances de tenue mécanique et d'isolation électrique des "traversées" décrites plus haut (passages hermétiques et électriquement isolés d'une liaison électrique au travers d'une paroi métallique) pour la réalisation d'un capteur dans lequel l'intégralité de la structure est réalisée en un matériau métallique, très avantageusement un matériau parfaitement biocompatible tel que le titane.

Essentiellement, le capteur de l'invention est réalisé à partir de deux substrats distincts de ce matériau métallique lors d'étapes préliminaires de réalisation des divers éléments portés par chacun des deux substrats, selon des technologies de fabrication en elles-mêmes conventionnelles.

En particulier, chacun des deux substrats porte un élément de transducteur respectif, typiquement une électrode et une contre-électrode en vis-à-vis : l'un des substrats comprend la région opérante déformable (membrane ou autre) solidaire du premier élément de transducteur (l'électrode), tandis que l'autre substrat porte l'autre élément de transducteur (la contre-électrode), qui est fixe, de manière à faire varier entre ces deux éléments un paramètre électrique qui pourra être transformé ensuite en signal de capteur.

Dans cette configuration, l'électrode et la contre-électrode sont reliées à deux bornes respectives du capteur, qui sont deux régions de l'un ou l'autre substrat en continuité électrique avec l'électrode et la contre-électrode.

L'isolation entre ces deux bornes de capteur est obtenue par la technologie d'isolation verticale de traversée décrite plus haut : il s'agit de réaliser, sur l'un ou l'autre des substrats (ou sur les deux) une interface comprenant au moins une couche latérale périphérique permettant d'isoler les deux bornes du capteur, qui sont chacune en continuité électrique avec l'un des éléments de transducteur respectifs.

Cette configuration électrode/contre-électrode n'est toutefois pas limitative, et d'autres configurations de transducteurs son envisageables, notamment, comme on le décrira plus bas, l'utilisation d'une couche piézoélectrique produisant entre ses faces un potentiel électrique variable en fonction de la contrainte mécanique qui lui est appliquée. Dans ce cas, les deux faces de la couche sont reliées à des bornes respectives du capteur, et l'interface de la couche latérale périphérique a pour fonction d'isoler entre elles les deux bornes et leurs liaisons à la lame piézoélectrique.

La Figure 15 illustre un exemple de capteur réalisé suivant les enseignements de l'invention.

Le capteur 80 comporte, essentiellement, deux substrats distincts 100 et 200, typiquement réalisés en un matériau biocompatible, biostable et résistant à la corrosion tel que le titane.

Le substrat 100 comporte une face externe 102 et une face interne 104, et le second substrat comporte une face externe 202 et une face interne 204.

Les faces 104 et 204 sont disposées en vis-à-vis, tandis que la face externe 102 du substrat 100 reçoit directement une sollicitation, par exemple une pression P d'un fluide environnant en contact avec la face externe 202, qui a pour effet de déformer un élément central 106 du substrat entouré par une fine membrane périphérique 108 déformable. Avantageusement, dans ce mode de réalisation ainsi dans ceux qui seront décrits par la suite, si la face externe 102 du substrat 100 est directement exposée à un fluide corporel tel que le sang (par exemple dans le cas d'un capteur de dispositif implantable *in situ* logé dans une cavité du cœur), cette face externe 102 peut faire l'objet d'une microstructuration ou nanostructuration de surface, afin de réduire à cet endroit les phénomènes de colonisation cellulaire susceptibles de modifier sur le long terme les performances du capteur.

La partie centrale 106, ci-après également désignée "région opérante" du substrat 100, comporte sur la face interne 104 du substrat un premier élément de transducteur, typiquement une électrode 110, qui se déplace en direction transversale au gré des sollicitations variables, par exemple des variations de la pression P subie sur la face externe 102 dans la région entourant la partie amincie déformable 108.

Le substrat 200 comprend une région 206, ci-après également désignée "région opérante" du substrat 200. Cette région opérante est située en vis-à-vis de la partie centrale 106 du substrat 100 et elle comporte un second élément de transducteur 210 tel qu'une contre-électrode.

On notera que les électrodes 110 et/ou 210 peuvent être des électrodes rapportées ou, en variante, constituées par la surface elle-même du substrat 100 ou 200, si la conductivité de ce substrat est suffisante.

Les deux substrats 100 et 200 sont réunis par une liaison de solidarisation 300. Cette liaison peut être obtenue directement par mise en contact des deux substrats, avec adhésion par effet électrostatique (force de van der Waals) ou par soudure thermique, thermomécanique ou autre procédé conventionnel pour la soudure des wafers.

Il est également possible de prévoir pour la liaison 300 une couche intermédiaire en un matériau tiers tel que platine, oxyde de titane, nitrure de titane, or, alumine et des combinaisons des précédents.

L'électrode 110 et la contre-électrode 210 forment ensemble un transducteur 82, qui comporte deux pôles, l'un des pôles (l'électrode 110) étant relié à une première borne de capteur 84 et l'autre pôle (la contre-électrode 210) étant relié à une seconde borne de capteur 86.

Ces bornes 84, 86 étant en continuité électrique avec le matériau du substrat 100, il convient de les isoler entre elles, ce qui est obtenu par une interface comprenant une couche latérale périphérique 214 réalisée de la même manière, et avec les différentes variantes possibles, que la couche latérale périphérique 42 des diverses traversées décrites ci-dessus en référence aux Figures 1 à 8.

Ainsi, pour le capteur 80 de la Figure 15, la couche périphérique isolante 214 isole électriquement la région opérante centrale 206 du substrat 200 (à laquelle est reliée la première borne de capteur 86) du reste 212 du substrat (auquel est reliée la seconde borne de capteur 84), tout en assurant une parfaite solidarisation mécanique des deux régions 206 et 212 du substrat 200.

On notera que dans l'exemple illustré Figure 15, ainsi que sur les figures suivantes, l'interface avec la couche latérale périphérique 214 est réalisée sur le substrat 200. Mais cette interface pourrait aussi être, en variante ou en complément, réalisée sur le substrat 100, dès lors qu'il s'agit d'assurer une isolation fiable et efficace entre les deux éléments de transducteur, en l'espèce l'électrode 110 et la contre-électrode 210.

On notera par ailleurs qu'une isolation qui ne serait basée que sur une couche de scellement intercalaire 300 isolante ne serait pas suffisante pour obtenir les performances voulues. En effet, cette couche de scellement 300 étant très mince, dans la pratique des courts-circuits peuvent s'établir, notamment du fait de pollutions extérieures liées au procédé d'usinage (en particulier lors de la découpe des puces) et/ou de conditions d'utilisation, où des impuretés ou des cristaux conducteurs peuvent créer un pont électrique entre les deux substrats.

La variation de l'intervalle d entre les deux éléments du transducteur 82 (l'électrode 110 et la contre-électrode 210) peut être mesurée par différentes techniques en elles-mêmes connues :
- mesure capacitive entre l'électrode 110 et la contre-électrode 210 (sans contact entre ces électrodes, ou bien avec contact mécanique, l'électrode 110 pouvant venir en butée contre la contre-électrode 210 : dans ce dernier cas une couche isolante supplémentaire peut être ajoutée sur l'une ou l'autre des électrodes) ;
- mesure résistive entre l'électrode 110 et la contre-électrode 210, l'électrode mobile 110 venant successivement en contact avec une série de contre-électrodes en étoile ou concentriques, fixes, formées sur le substrat 200, de manière à établir des ponts électriques : la déformation de la membrane 108 entraine un contact physique entre électrode et contre-électrodes, avec un nombre variable de ponts électriques en fonction de la contrainte extérieure subie ;
- mesure piézoélectrique, grâce à une couche piézoélectrique déposée sur la surface interne de la membrane 108: la déformation de la membrane engendre entre les deux faces de la couche piézoélectrique un potentiel électrique variable que l'on peut recueillir via un simple contact électrique pris au niveau du substrat métallique en continuité avec la couche électrique. Dans ce cas, le transducteur ne comporte plus de contre-électrode. Mais pour mesurer une différence de potentiel entre les deux faces de la couche piézoélectrique déposée sur la face interne du substrat, deux contacts correspondants sont nécessaires, avec un contact tel que décrit plus haut (pour la face de la couche piézoélectrique en contact avec la face interne du substrat) et un contact sur la face opposée, libre de la couche piézoélectrique, tournée vers l'intérieur du dispositif.

La Figure 16 illustre une variante dans laquelle le substrat 100 comprend un évidement 112 non traversant, dont le fond constitue la membrane déformable 108 qui est dans ce cas située au niveau de la face interne 104 du substrat 100.

Dans le cas d'un capteur accélérométrique ou microphonique, l'évidement 112 peut loger une masse inertielle rapportée 114 permettant d'accroitre la sensibilité du capteur aux sollicitations externes. Le report d'une masse inertielle, plus lourde donc en général en matériau non biocompatible tel le tungstène, peut aussi (et préférentiellement) se faire à partir de la configuration de la Figure 15, où la membrane 108 est complètement circulaire (pas de "bosse" au niveau de l'îlot 106 en titane) et l'élément rapporté est assemblé sur cette membrane, tout en restant ainsi logé au sein du dispositif, de manière à ne pas être soumis à des contraintes de biocompatibilité.

La membrane peut être de toute géométrie connue circulaire, elliptique, rectangulaire, quelconque, plate ou structurée, etc.

Dans tous les cas, la structure de l'invention permet d'intégrer complètement au capteur 80 les éléments du transducteur 82, avec des dimensions très faibles et sans qu'il soit nécessaire de prévoir un conditionnement protecteur externe biocompatible additionnel.

Le capteur ainsi réalisé peut être soit un composant autonome (auquel cas il sera associé à une source d'alimentation intégrée ou distante), soit un élément d'un dispositif implantable plus complexe, par exemple un capteur intégré à la surface du boitier d'un dispositif implantable, ou encore être un composant reporté dans une sonde de détection/stimulation cardiaque, bénéficiant ainsi de l'alimentation du dispositif.

La Figure 17 illustre la possibilité d'incorporer au capteur de l'invention un composant électronique additionnel 400 tel qu'une puce électronique.

La structure de base est la même que celle décrite plus haut et illustrée

Figures 15 ou 16, et le composant est logé dans une cavité réalisée dans le substrat 200 (comme la cavité 216 illustrée sur la Figure 17), ou bien dans le substrat 100 selon le même principe.

Le matériau de la puce, généralement le silicium, n'est pas un matériau biocompatible mais la puce sera entièrement enfermée entre les deux substrats 100 et 200 qui, eux, sont réalisés en un matériau biocompatible tel que le titane.

Le composant 400 est relié à des plages de connexion 222, 224, formées au fond de la cavité 216 du substrat 200. Ces plages de connexion du substrat 200 sont reliées par des conducteurs respectifs 402, 404 aux bornes du composant 400 par des technologies connues telles que typiquement un câblage filaire de type *wire bonding.*

Outre l'interface comprenant la couche latérale périphérique 214, il convient de créer au moins une autre interface comprenant une couche latérale périphérique supplémentaire 218, réalisée selon les mêmes enseignements que la couche 214. Cette interface supplémentaire crée une isolation entre i) une région supplémentaire isolée 220 et ii) la région opérante 206 reliée à la première borne de capteur 86 ainsi que iii) le reste du substrat 212 relié à la seconde borne de capteur 84. Une troisième borne de capteur 88 est reliée à cette région supplémentaire isolée 220, pour réaliser une liaison à la puce de composant 400.

Sur les figures, les flèches désignent des régions où sont réalisées les prises de contact des première et seconde bornes de capteur 84 et 86 (et de la troisième borne 88 dans le cas de la Figure 17).

La technique proposée par l'invention permet d'effectuer une prise de contact direct, en soudant ou sertissant un élément métallique tel que fil, ruban ou bague sur des zones correspondantes de l'un et/ou l'autre des substrats 100 et 200

Il convient de réaliser ces prises de contact sur des zones qui ne sont pas susceptibles d'induire de contraintes mécaniques significatives sur le capteur, qui pourraient perturber la sensibilité de celui-ci par une déformation permanente difficile à contrôler.

En particulier, le contact de la borne de capteur 84 avec le reste du substrat 212 hors de la zone opérante 206 peut être réalisé non seulement, comme illustré, par la masse externe 202 du substrat 200, mais également par la tranche de celui-ci, comme en 84', ou même par la tranche du substrat 100, comme en 84", dès lors que la liaison de solidarisation 300 entre les deux substrats est une liaison suffisamment conductrice pour assurer la continuité électrique voulue.

Dans tous les cas, une métallisation peut être rapportée sur la zone du substrat à contacter pour faciliter la prise de contact, par exemple par dépôt d'une pastille ou d'une couche métallique en un matériau tel qu'or, platine, palladium, niobium, iridium ou toutes combinaisons de ces matériaux.

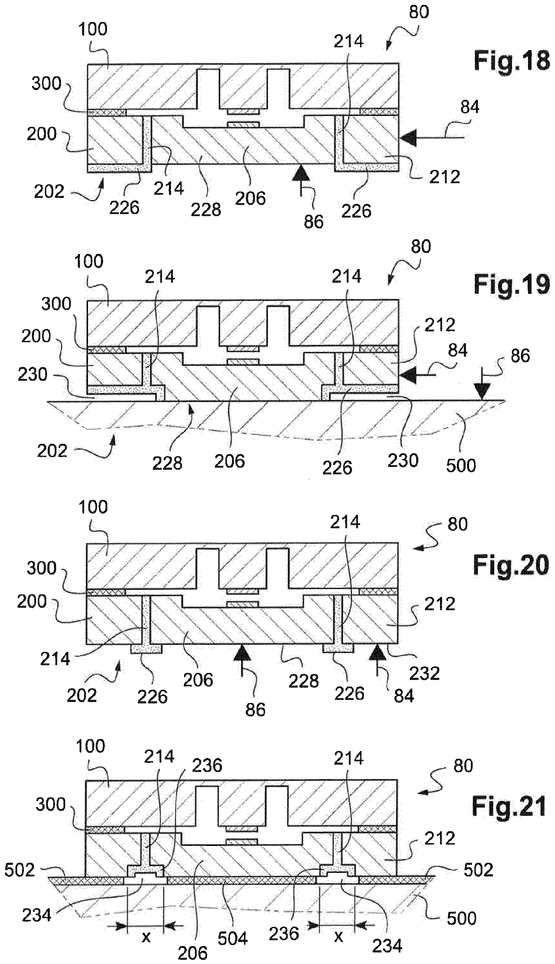

La Figure 18 illustre un perfectionnement de la structure de capteur de la Figure 15 (ce perfectionnement étant également applicable à la structure de la Figure 16), dans lequel une couche isolante d'oxyde 226 a été sélectivement déposée sur une face externe d'un substrat, la face externe 202 du substrat 200 dans l'exemple illustré.

Cette couche isolante 226, réalisée suivant les mêmes techniques que celle décrite plus haut en relation avec les Figures 1 à 14, permet de délimiter une première zone de contact à nu 228 destinée à la prise de contact d'une première borne 86 du capteur. Cette zone est parfaitement séparée et isolée de la zone du reste du substrat 212 sur laquelle est réalisé le contact avec l'autre borne de capteur, par exemple sur la tranche du substrat 200.

La couche extérieure isolante d'oxyde 226 prolonge en surface la couche interne latérale périphérique 214 et est obtenue, de la même façon, par oxydation du métal constituant le substrat 200 : dans l'exemple décrit, une oxydation du titane Ti en oxyde de titane TiO₂.

La Figure 19 illustre une variante du capteur de la Figure 18 dans laquelle le substrat 200 comprend sur sa face externe 202 une zone en retrait 230, gravée lors des étapes de micro-usinage du substrat. Cette zone 230 permet notamment de réaliser le contact avec la zone 228 de la partie opérante centrale 206 du substrat par report du capteur sur un support ou autre substrat conducteur 500, qui constitue un pôle relié à l'une des bornes 86 du capteur. L'autre borne 84 est par exemple réalisée par une prise de connexion sur la tranche du substrat 200.

La Figure 20 illustre une variante du capteur de la Figure 18 permettant de réaliser sur une seule et même face du substrat 200 (la face externe 202) des prises de contact pour les bornes des capteurs 84 et 86. Ceci peut être intéressant notamment lorsque le capteur est intégré dans un dispositif médical implantable actif de taille réduite tel qu'un neurostimulateur de très faible épaisseur, ou un stimulateur cardiaque sans sonde de type dit *leadless,* directement implanté dans une cavité du cœur.

L'étendue de la couche extérieure isolante d'oxyde 226 est réduite de manière à laisser à nu une deuxième zone de contact 232 au-dessus de la région 212 du reste du substrat. On peut ainsi réaliser une prise de connexion depuis la face externe 202 du substrat 200 à la fois vers la borne de capteur 86 (par la première zone de contact 228 décrite plus haut Figure 18) et vers l'autre borne de capteur 84 (par la deuxième zone de contact mise à nu 232).

La Figure 21 illustre une variante du capteur de la Figure 19, dans laquelle des zones en retrait 234 sont formées dans la face externe du substrat 200 au voisinage de la couche interne latérale périphérique 214, en laissant à nu cette même face dans la région de l'îlot central 206 et dans la région 212 du reste du substrat, et avec les parois des zones en retrait 234 couvertes par la couche isolante d'oxyde 236. Cette configuration permet en particulier de réaliser des prises de contact depuis une même face (la face externe) du substrat 200, par exemple lorsque le capteur 80 est reporté sur un support ou autre substrat conducteur 500 comportant des plages conductrices 502 et 504 qui venant directement en contact avec les régions respectives 206 et 212 du substrat 200. On notera par ailleurs que la zone d'isolation *x* entre les deux bornes du capteur est aisément modulable par un choix approprié de l'étendue de la couche d'oxyde formée en 236 sur les parois des zones en retrait 234, l'oxyde pouvant si nécessaire déborder sur le substrat hors de la cavité pour augmenter la distance d'isolation *x*.

## Revendications

1. Un capteur (80) comprenant :
- un premier substrat (100) en matériau conducteur avec une face externe (102) et une face interne (104), comprenant :
• une région opérante (106) déformable sous l'effet d'une sollicitation mécanique (P) appliquée sur la face externe, et
• sur la face interne de la région opérante, un premier élément de transducteur (110), mobile sous l'effet de la déformation de la région opérante ;
- un second substrat (200) en matériau conducteur avec une face externe (202) et une face interne (204) tournée vers la face interne du premier substrat, et comprenant une région opérante (206) ;
- un second élément de transducteur (210) apte à coopérer avec le premier élément de transducteur (110) pour faire varier un paramètre électrique entre le premier et le second élément de transducteur sous l'effet de ladite sollicitation mécanique ;
- une liaison (300) de solidarisation du premier substrat au second substrat, située à l'extérieur des régions opérantes ;
- une première borne de capteur (84), électriquement couplée au premier élément de transducteur (110) ; et
- une seconde borne de capteur (86), électriquement couplée au second élément de transducteur (210), dans lequel, pour au moins l'un (200) des substrats :
- la région opérante (206) s'étend en direction transversale dans l'épaisseur du substrat (200) d'une face à l'autre de celui-ci ;
- la région opérante (206) est en forme d'ilot de contour fermé, physiquement et électriquement isolé du reste du substrat, l'une des bornes de capteur (86) étant électriquement reliée à l'ilot ;
- il est prévu entre la région opérante (206) et le reste (212) du substrat une interface comprenant au moins une couche latérale périphérique (214) en matériau électriquement isolant propre à assurer à la fois un assujettissement mécanique de la région opérante au substrat et une isolation électrique entre la région opérante et le substrat,
la couche latérale périphérique (214) étant monolithique avec le reste du substrat (212) et avec la région opérante (206), enveloppant en direction latérale la région opérante (206) sur toute sa périphérie, et s'étendant en direction transversale sur l'épaisseur du substrat (200).

2. Le capteur de la revendication 1, dans lequel le matériau du premier (100) et du second substrat (200) est un matériau appartenant à la classe des matériaux biocompatibles, biostables et résistants à la corrosion.

3. Le capteur de la revendication 2, dans lequel le matériau biocompatible, biostable et résistant à la corrosion est le titane.

4. Le capteur de la revendication 1, dans lequel le matériau électriquement isolant de la couche latérale périphérique (214) est un oxyde du matériau du substrat métallique.

5. Le capteur de la revendication 1, dans lequel la région opérante déformable (106) du premier substrat (100) est située sur une partie d'épaisseur réduite (112) du premier substrat.

6. Le capteur de la revendication 1, dans lequel :
- la région opérante (206) du second substrat est une région située en vis-à-vis de la région opérante (106) déformable du premier substrat ;
- le second élément de transducteur (210) est un élément disposé sur la face interne de la région opérante (206) du second substrat en vis-à-vis du premier élément de transducteur (110) ; et
- les premier et second éléments de transducteur (110, 210) sont des éléments de l'un parmi : un transducteur capacitif, un transducteur résistif, et un transducteur inductif.

7. Le capteur de la revendication 1, dans lequel :
- la région opérante (106) déformable du premier substrat comprend une couche piézoélectrique ; et
- les premier et second éléments de transducteur (110, 210) sont des surfaces opposées de la couche piézoélectrique, comprenant une surface en contact avec la face interne de l'un des substrats, et une surface libre tournée à l'intérieur du capteur vers la face interne de l'autre substrat.

8. Le capteur de la revendication 1, dans lequel :
- le capteur comporte en outre un composant électronique (400) incorporé au capteur et comprenant une première borne de composant et une seconde borne de composant ; et
- ledit au moins un (200) des substrats comprend :
• sur sa face interne, un évidement (216) logeant le composant électronique ;
• une autre interface, comportant au moins une autre couche latérale périphérique (218) définissant un autre ilot (220) isolé de l'ilot (206) et du reste (212) du substrat ; et
• une liaison électrique (402) de la première borne de composant à l'autre ilot (220), et une liaison électrique (404) de la seconde borne de composant au reste du substrat (212) ou à l'ilot.

9. Le capteur de la revendication 1, dans lequel la liaison de solidarisation est une liaison soudée des premier et second substrats entre eux.

10. Le capteur de la revendication 1, dans lequel la liaison de solidarisation comprend entre le premier substrat et le second substrat une couche intercalaire (300) de scellement.

11. Le capteur de la revendication 10, dans lequel le matériau de la couche intercalaire de scellement (300) est un matériau électriquement conducteur apte à assurer une continuité électrique entre le premier et le second substrat.

12. Le capteur de la revendication 1, dans lequel ledit au moins un (200) des substrats comprend sur sa face externe (202) une couche isolante de surface (226), avec dans la région de l'ilot (206) au moins une zone de contact (228) mise à nu ou laissée à nu.

13. Le capteur de la revendication 12, dans lequel la zone de contact (228) est proéminente en direction transversale par rapport au reste de la face externe du substrat.

14. Le capteur de la revendication 12, comprenant :
- deux zones de contact avec deux zones proéminentes respectives mises à nu ou laissées à nu, l'une dans la région de l'îlot (206) et l'autre dans la région du reste du substrat (212), et
- entre les deux zones proéminentes, dans la région de la couche interne latérale périphérique (214), une zone en retrait (234) avec une couche isolante (236).

## Patentansprüche

1. Sensor (80), umfassend:
- ein erstes Substrat (100) aus einem leitenden Material mit einer äußeren Seite (102) und einer inneren Seite (104), umfassend:
• einen wirksamen Bereich (106), der unter der Einwirkung einer mechanischen Beanspruchung (P), die auf die äußere Seite aufgebracht wird, verformbar ist, und
• auf der inneren Seite des wirksamen Bereichs, ein erstes Wandlerelement (110), das unter der Einwirkung der Verformung des wirksamen Bereichs beweglich ist;
- ein zweites Substrat (200) aus einem leitenden Material mit einer äußeren Seite (202) und einer inneren Seite (204), die zu der inneren Seite des ersten Substrats hin weist, und das einen wirksamen Bereich (206) umfasst;
- ein zweites Wandlerelement (210), das zum Zusammenwirken mit dem ersten Wandlerelement (110) imstande ist, um einen elektrischen Parameter zwischen dem ersten und dem zweiten Wandlerelement unter der Einwirkung der mechanischen Beanspruchung zu verändern;
- eine feste Verbindung (300) des ersten Substrats mit dem zweiten Substrat, die sich außerhalb der wirksamen Bereiche befindet;
- eine erste Sensorklemme (84), die elektrisch mit dem ersten Wandlerelement (110) gekoppelt ist; und
- eine zweite Sensorklemme (86), die elektrisch mit dem zweiten Wandlerelement (210) gekoppelt ist,
wobei, bei mindestens dem einen Substrat (200) der Substrate:
- der wirksame Bereich (206) sich in Querrichtung in der Dicke des Substrats (200) von einer Seite zu der anderen von diesem letzten erstreckt;
- der wirksame Bereich (206) die Form einer Insel mit geschlossener Kontur aufweist, die von dem Rest des Substrats physikalisch und elektrisch isoliert ist, wobei eine der Sensorklemmen (86) mit der Insel elektrisch verbunden ist;
- zwischen dem wirksamen Bereich (206) und dem Rest (212) des Substrats eine Schnittstelle vorgesehen ist, die mindestens eine umlaufende seitliche Schicht (214) aus einem elektrisch isolierenden Material umfasst, die imstande ist, gleichzeitig eine mechanische Befestigung des wirksamen Bereichs an dem Substrat und eine elektrische Isolierung zwischen dem wirksamen Bereich und dem Substrat sicherzustellen,
wobei die umlaufende seitliche Schicht (214) mit dem Rest des Substrats (212) und mit dem wirksamen Bereich (206) monolithisch ist, in seitlicher Richtung den wirksamen Bereich (206) über dessen gesamten Umfang umgibt und sich in Querrichtung über die Dicke des Substrats (200) erstreckt.

2. Sensor nach Anspruch 1, wobei das Material des ersten Substrats (100) und des zweiten Substrats (200) ein Material ist, das zu der Gattung der biokompatiblen, biostabilen und korrosionsbeständigen Materialien gehört.

3. Sensor nach Anspruch 2, wobei das biokompatible, biostabile und korrosionsbeständige Material Titan ist.

4. Sensor nach Anspruch 1, wobei das elektrisch isolierende Material der umlaufenden seitlichen Schicht (214) ein Oxid des Materials des metallischen Substrats ist.

5. Sensor nach Anspruch 1, wobei sich der verformbare wirksamen Bereich (106) des ersten Substrats (100) auf einem Teil mit reduzierter Dicke (112) des ersten Substrats befindet.

6. Sensor nach Anspruch 1, wobei:
- der wirksame Bereich (206) des zweiten Substrats ein Bereich ist, der sich dem verformbaren wirksamen Bereich (106) des ersten Substrats gegenüber befindet;
- das zweite Wandlerelement (210) ein Element ist, das auf der inneren Seite des wirksamen Bereichs (206) des zweiten Bereichs dem ersten Wandlerelement (110) gegenüber angeordnet ist; und
- das erste und zweite Wandlerelement (110, 210) Elemente von einem aus: einem kapazitiven Wandler, einem resistiven Wandler und einem induktiven Wandler sind.

7. Sensor nach Anspruch 1, wobei:
- der verformbare wirksame Bereich (106) des ersten Substrats eine piezoelektrische Schicht umfasst; und
- das erste und zweite Wandlerelement (110, 210) gegenüberliegende Oberflächen der piezoelektrischen Schicht sind, die eine Oberfläche in Kontakt mit der inneren Seite des einen der Substrate und eine freie Oberfläche, die im Inneren des Sensors zu der inneren Seite des anderen Substrats hin weist, umfassen.

8. Sensor nach Anspruch 1, wobei:
- der Sensor ferner ein elektronisches Bauteil (400) aufweist, das in den Sensor eingebaut ist und eine erste Bauteilklemme und eine zweite Bauteilklemme umfasst; und
- das mindestens eine Substrat (200) der Substrate Folgendes umfasst:
• auf seiner inneren Seite, eine Ausnehmung (216), die das elektronische Bauteil aufnimmt;
• eine weitere Schnittstelle, die mindestens eine weitere umlaufende seitliche Schicht (218) aufweist, die eine weitere Insel (220) definiert, die von der Insel (206) und dem Rest (212) des Substrats isoliert ist; und
• eine elektrische Verbindung (402) der ersten Bauteilklemme mit der weiteren Insel (220) und eine elektrische Verbindung (404) der zweiten Bauteilklemme mit dem Rest des Substrats (212) oder mit der Insel.

9. Sensor nach Anspruch 1, wobei die feste Verbindung eine Schweißverbindung des ersten und zweiten Substrats miteinander ist.

10. Sensor nach Anspruch 1, wobei die feste Verbindung zwischen dem ersten Substrat und dem zweiten Substrat eine Versiegelungszwischenschicht (300) umfasst.

11. Sensor nach Anspruch 10, wobei das Material der Versiegelungszwischenschicht (300) ein elektisch leitendes Material ist, das imstande ist, einen Stromdurchgang zwischen dem ersten und dem zweiten Substrat sicherzustellen.

12. Sensor nach Anspruch 1, wobei das mindestens eine Substrat (200) der Substrate auf seiner äußeren Seite (202) eine isolierende Oberflächenschicht (226) umfasst, mit im Bereich der Insel (206) mindestens einer Kontaktzone (228), die freigelegt ist oder blank belassen ist.

13. Sensor nach Anspruch 12, wobei die Kontaktzone (228) in Querrrichtung gegenüber dem Rest der äußeren Seite des Substrats eine Erhebung bildet.

14. Sensor nach Anspruch 12, umfassend:
- zwei Kontaktzonen mit zwei jeweiligen Erhebungen bildenden Zonen, die freigelegt sind oder blank belassen sind, die eine im Bereich der Insel (206) und die andere im Bereich des Restes des Substrats (212), und
- zwischen den zwei Erhebungen bildenden Zonen, im Bereich der umlaufenden seitlichen inneren Schicht (214), eine zurückgezogene Zone (234) mit einer isolierenden Schicht (236).

## Claims

1. A sensor (80) comprising:
- a first substrate (100) made of conductive material with an outer side (102) and an inner side (104), comprising:
• an operative region (106) deformable under the effect of a mechanical stress (P) applied to the outer side, and
• on the inner side of the operative region, a first transducer element (110), mobile under the effect of the operative region deformation;
- a second substrate (200) made of a conductive material with an outer side (202) and an inner side (204) directed towards the inner side of the first substrate, and comprising an operative region (206);
- a second transducer element (210) adapted to cooperate with the first transducer element (110) to vary an electrical parameter between the first and the second transducer element under the effect of said mechanical stress;
- a connection (300) for fastening the first substrate to the second substrate, located out of the operative regions;
- a first sensor terminal (84), electrically coupled to the first transducer element (110); and
- a second sensor terminal (86), electrically coupled to the second transducer element (210),
wherein, for at least one (200) of the substrates:
- the operative region (206) extends in transverse direction in the thickness of the substrate (200), from one side to the other one of the latter;
- the operative region (206) is in the form of a closed-contour islet, physically and electrically isolated from the rest of the substrate, one of the sensor terminals (86) being electrically connected to an islet;
- between the operative region (206) and the rest (212) of the substrate, an interface is provided that comprises at least one peripheral lateral layer (214) made of an electrically isolating material able to ensure both a mechanical fastening of the operative region to the substrate and an electrical insulation between the operative region and the substrate,
the peripheral lateral layer (214) being single-piece with the rest (212) of the substrate and with the operative region (206), enveloping in the lateral direction the operative region (206) over the whole periphery thereof, and extending in transverse direction over the thickness of the substrate (200).

2. The sensor of claim 1, wherein the material of the first (100) and the second (200) substrate is a material belonging to the class of the biocompatible, biostable and corrosion-resistant materials.

3. The sensor of claim 2, wherein the biocompatible, biostable and corrosion-resistant material is titanium.

4. The sensor of claim 1, wherein the electrically isolating material of the peripheral lateral layer (214) is an oxide of the material of the metallic substrate.

5. The sensor of claim 1, wherein the deformable operative region (106) of the first substrate (100) is located on a reduced-thickness portion (112) of the first substrate.

6. The sensor of claim 1, wherein:
- the operative region (206) of the second substrate is a region located opposite the deformable operative region (106) of the first substrate;
- the second transducer element (210) is an element arranged on the inner side of the operative region (206) of the second substrate opposite the first transducer element (110); and
- the first and second transducer elements (110, 210) are elements of one among: a capacitive transducer, a resistive transducer and an inducive transducer.

7. The sensor of claim 1, wherein:
- the deformable operative region (106) of the first substrate comprises a piezoelectric layer; and
- the first and second transducer elements (110, 210) are opposite surfaces of the piezoelectric layer, comprising a surface in contact with the inner side of one of the substrates, and a free surface directed inside of the sensor towards the inner side of the other substrate.

8. The sensor of claim 1, wherein:
- the sensor further includes an electronic component (400) incorporated to the sensor and comprising a first component terminal and a second component terminal; and
- said at least one (200) of the substrates comprises:
• on its inner side, a recess (216) housing the electronic component;
• another interface, including at least another peripheral lateral layer (218) defining another islet (220) isolated from the islet (206) and from the rest (212) of the substrate; and
• an electric connection (402) of the first component terminal to the other islet (220), and an electric connection (404) of the second component terminal to the rest (212) of the substrate or to the islet.

9. The sensor of claim 1, wherein the fastening connection is a welded connection of the first and second substrates to each other.

10. The sensor of claim 1, wherein the fastening connection comprises, between the first substrate and the second substrate, an intermediate sealing layer (300).

11. The sensor of claim 10, wherein the material of the intermediate sealing layer (300) is an electrically conductive material adapted to ensure an electrical continuity between the first and the second substrate.

12. The sensor of claim 1, wherein said at least one (200) of the substrates comprises on its outer side (202) an insulating surface layer (226), with, in the region of the islet (206), at least one bared or left-bare contact area (228).

13. The sensor of claim 12, wherein the contact area (228) is prominent in transverse direction with respect to the rest of the outer side of the substrate.

14. The sensor of claim 12, comprising:
- two contact areas with two respective bared or left-bare prominent areas, one in the region of the islet (206) and the other in the region of the rest (212) of the substrate, and
- between the two prominent areas, in the region of the peripheral lateral inner layer (214), a set-back area (234) with an isolating layer (236).
